# EUROPÄISCHE PATENTSCHRIFT

(11) **EP 0 793 953 B1**
(45) Veröffentlichungstag und Bekanntmachung des Hinweises auf die Patenterteilung: **07.05.2003**
(21) Anmeldenummer: 97102111.8
(22) Anmeldetag: 11.02.1997
(51) Int. Cl.: A61F 13/62, A61F 13/15

(54) **Wegwerfwindel zum einmaligen Gebrauch und Verfahren zu deren Herstellung**
Disposable diaper and its method of manufacturing
Couche à usage unique et son procédé de fabrication

(30) Priorität: 07.03.1996 DE 19608857; 31.01.1997 DE 19703557
(43) Veröffentlichungstag der Anmeldung: 10.09.1997
(73) Patentinhaber: Paul Hartmann Aktiengesellschaft, 89522 Heidenheim (DE)
(72) Erfinder: Bitterhof, Andreas, Dr., 71229 Leonberg (DE); Denk, Bettina, 89075 Ulm (DE); Oltmann, Eckhard, Dr., 89522 Heidenheim (DE)
(74) Vertreter: Dreiss, Fuhlendorf, Steimle & Becker

(56) Entgegenhaltungen:
- EP-A- 0 496 567
- EP-A- 0 529 681
- EP-A- 0 719 532
- WO-A-83/03754
- WO-A-92/22274
- US-A- 3 981 763
- US-A- 5 399 219

## Beschreibung

Die Erfindung betrifft eine Wegwerfwindel zum einmaligen Gebrauch gemäß dem Oberbegriff des Patentanspruchs 1, sowie ein Verfahren zu deren Herstellung.

Aus der EP 0 321 232 B1 ist eine gattungsgemäße Wegwerfwindel nach Art einer Höschenwindel bekannt, die erste Verschlußteile an den Längsrändern des rückwärtigen Endbereichs und zweite Verschlußteile in dem vorderen Endbereich aufweist, die mit den ersten verbindbar sind, zum Festlegen der Windel an einer Person. Dazu weisen die ersten Verschlußteile Häkchen auf, die mit Ösen der zweiten Verschlußteile in Eingriff bringbar sind. Darüber hinaus weisen die ersten Verschlußteile einen Klebstoffbereich auf, der insbesondere dazu dient, die gebrauchte Windel in zusammengerolltem Zustand mittels der ersten Verschlußteile zu fixieren.

Bei der Herstellung derartiger Wegwerfwindeln werden die ersten Verschlußteile an der im flachgelegten Zustand befindlichen Windel befestigt, wobei die Verschlußteile dann von den Längsrändern abragen. Da die Maschinen sehr schnell laufen - es werden beispielsweise auf einer Maschine bis zu 600 Windeln pro Minute gefertigt - kann es durch die abragenden Verschlußteile zu technischen Schwierigkeiten kommen, da sie fähnchenartig auf der schnell laufenden Maschine flattern werden.

Zur Vermeidung dieser Probleme kann der Klebstoffauftrag dazu verwendet werden, die ersten Verschlußteile bereits bei der Herstellung und auch bei Nichtgebrauch der Windel in nach innen gefalteter Konfiguration zu fixieren.

Nachteilig an dieser bekannten Wegwerfwindel ist, daß der Aufbau der ersten Verschlußteile sehr aufwendig ist, da einerseits mechanische Verschlußelemente, wie die Häkchen, und andererseits ein Klebstoffbereich vorgesehen ist. Darüber hinaus kann der Klebstoffauftrag, beispielsweise durch Öl oder Puder, verschmutzen und ist dann im wesentlichen wirkungslos.

Klettverschlüsse als Verschlußmittel für Windeln sind beispielsweise auch aus der WO 92/09254 bekannt. Diese Druckschrift beschreibt jedoch eine textile, waschbare Windel. Textile Windeln werden gänzlich anders hergestellt als Wegwerfwindeln und die vorbekannte textile Windel weist keine separaten, von Längsrändern abragende Verschlußteile auf, so daß die vorgenannten produktionstechnischen Probleme der Wegwerfwindelherstellung hier nicht auftreten können. Diese textile Windel zeigt einen zusätzlichen Klettflausch, der dazu dient, die Häkchen der Klettverschlußteile beim Waschen der Windel zu schützen.

EP-A-0 496 567 beschreibt eine textile zur mehrfachen Verwendung bestimmte Windel mit einem vorderen und einem rückwärtigen Endbereich, die beide durch Längs- und Querränder der Windel begrenzt sind, mit separaten ersten Verschlussteilen, die eine erste Art mechanische Verschlusselemente aufweisen und links und rechts in dem rückwärtigen Endbereich befestigt sind und in Gebrauchsstellung von dem jeweiligen Längsrand abragen, sowie mit wenigstens einem zweiten Verschlussteil, das in dem vorderen Endbereich befestigt ist und eine zweite Art mechanischer Verschlusselemente aufweist, die mit den Verschlusselementen der ersten Art lösbar in Eingriff bringbar sind, um die Endbereiche zum Festlegen der Windel an einer Person zu verbinden, wobei wenigstens ein Halteelement mit Verschlusselementen der zweiten Art in dem rückwärtigen Endbereich angeordnet ist, um die ersten Verschlusselemente zumindest bei der Herstellung der Windel in auf die Windel zurückgefalteter Konfiguration zu halten. Die ersten Verschlussteile sind durch eine Naht zusammen mit den Halteelementen auf die Windel aufgenäht, wobei die Naht eine Art Scharnier bildet und die Anordnung in eingefalteter Konfiguration ganz innerhalb des rückwärtigen Endbereichs liegt.

Es ist Aufgabe der Erfindung, eine Wegwerfwindel mit mechanischen Verschlußmitteln bereitzustellen, die einfacher aufgebaut ist und deren Verschlußteile zumindest während der Herstellung der Windel fixierbar sind, sowie ein Verfahren zur Herstellung dieser Windel anzugeben.

Diese Aufgabe wird gelöst durch eine gattungsgemäße Wegwerfwindel mit den kennzeichnenden Merkmalen des Patentanspruchs 1, sowie ein Verfahren gemäß Anspruch 8.

Aufgrund der erfindungsgemäß vorgesehenen Halteelemente mit mechanischen Verschlußelementen sind die ersten Verschlußteile, die von den Längsrändern der Windel abragen, in auf der Windel zurückgefalteter Konfiguration ohne Klebstoff fixierbar. Dadurch ragen die ersten Verschlußteile bei Nichtgebrauch der Windel und insbesondere bei der Herstellung und Verpackung nicht von den Längsrändern ab, wodurch die vorgenannten produktionstechnischen Schwierigkeiten vermieden sind. Ein zusätzlicher Klebstoffauftrag ist überflüssig und die erfindungsgemäße Wegwerfwindel, insbesondere ihre Verschlußteile weisen einen einfacheren Aufbau auf.

Vorteilhafterweise sind die mechanischen Verschlußelemente der Verschlußteile als Haken bzw. Ösen nach Art eines bekannten und bewährten Klettverschlusses ausgebildet.

In einfacher Weise kann das Haftelement auf einem Verankerungsteil des ersten Verschlußteils befestigt sein gemäß Anspruch 3. Dann kann das Halteelement auf dem Verankerungsteil vormontiert und in einem gemeinsamen Verfahrensschritt zusammen mit dem ersten Verschlußteil an der Windel befestigt werden. Das Herstellungsverfahren ist damit beschleunigt.

In einer Ausgestaltung der Erfindung gemäß Anspruch 4 ist eine Elastizität des Materials der Windel im Bereich des Halteelements, also in Seitenbereichen, weniger beeinträchtigt als in der Ausgestaltung gemäß Anspruch 3, da das Halteelement mit einem Abstand von dem ersten Verschlußteil befestigt ist.

Um die Beeinträchtigung der Elastizität der Windel in den Bereichen der ersten Verschlußteile weiter zu reduzieren, ist gemäß Anspruch 5 und/oder 6 vorgesehen, die ersten Verschlußteile wenigstens teilweise aus dehnbarem und/oder elastischem Material auszugestalten.

Wenn die ersten Verschlußteile aus einem relativ formstabilen Material gemäß Anspruch 7 bestehen, wird die auf die Windel zurückgefaltete Konfiguration verbessert beibehalten, so daß die Halteelemente nur eine geringe Haltekraft ausüben müssen.

Das erfindungsgemäße Verfahren gemäß Anspruch 8 zur Herstellung der erfindungsgemäßen Wegwerfwindel zeichnet sich dadurch aus, daß zunächst das oder die Halteelemente auf der Windel befestigt werden, und dann vom Längsrand abragende Abschnitte der ersten Verschlußteile umgeschlagen werden, wobei die Verschlußelemente der Verschlußteile mit den Verschlußelementen der Halteelemente in Eingriff gelangen, so daß die Halteelemente die Verschlußteile im weiteren Verfahrensablauf in auf die Windel zurückgefalteter Konfiguration halten. Dadurch sind die Verschlußteile fixiert und können keine produktionstechnischen Probleme verursachen.

In Ausgestaltung des erfindungsgemäßen Verfahrens können zunächst die Halteelemente und dann die ersten Verschlußteile oder umgekehrt an der Windel befestigt werden. In weiterer Ausgestaltung der Erfindung kann das Halteelement gleichzeitig mit den ersten Verschlußteilen auf der Windel befestigt werden, wodurch der Verfahrensablauf beschleunigt wird. Beispielsweise könnte dazu die Windel gemäß Anspruch 3 ausgebildet sein.

Im folgenden wird die Erfindung anhand eines Ausführungsbeispiels unter Bezugnahme auf die Zeichnung im einzelnen erläutert. In der Zeichnung zeigen:
- Figur 1: eine Ansicht der erfindungsgemäßen Wegwerfwindel;
- Figur 2: eine Draufsicht auf die Windel gemäß Figur 1 im flachgelegten Zustand;
- Figur 3a: einen Querschnitt eines Seitenbereichs der Windel mit einem ersten Verschlußelement entlang der Linie III-III aus Figur 2;
- Figur 3b: das Verschlußteil aus Figur 3a in gefaltetem Zustand;
- Figur 4a/4b: eine andere Ausführungsform der erfindungsgemäßen Windel in einer Darstellung wie Figuren 3a und b.

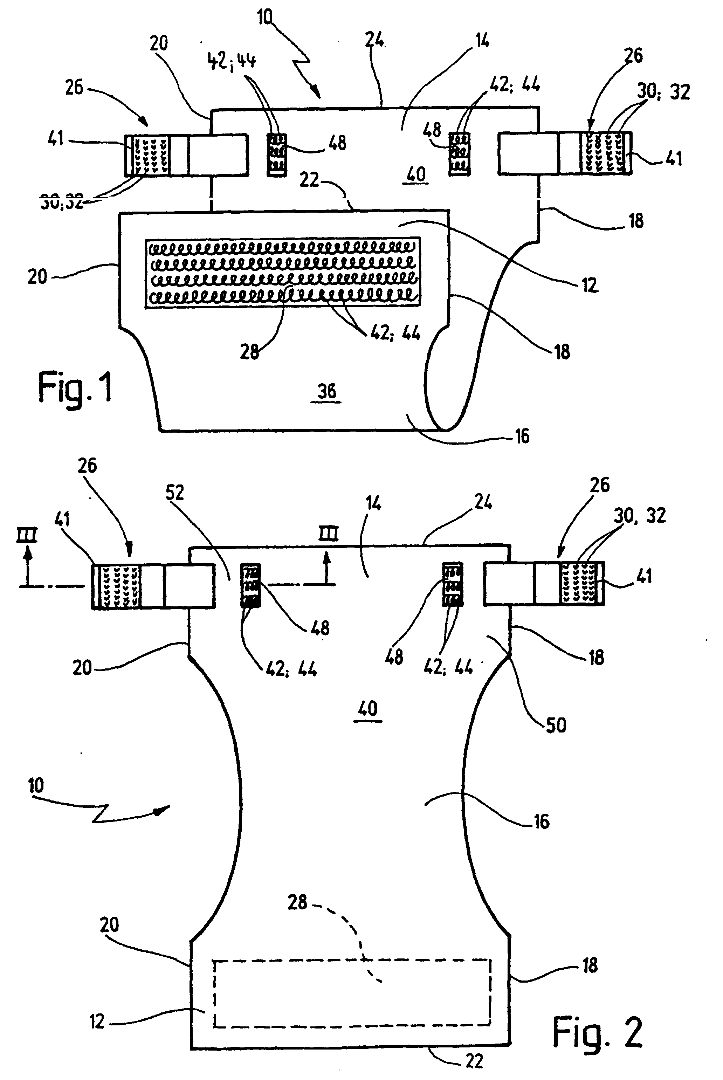

Eine in der Zeichnung dargestellte Wegwerfwindel 10 zum einmaligen Gebrauch kann eine Babywindel oder ein Wegwerfhygieneprodukt für inkontinente erwachsene Personen sein. Sie weist im flachgelegten Zustand (Figur 2) bevorzugt eine sanduhrförmige Form auf, mit vorderem Endbereich 12 und rückwärtigem Endbereich 14 sowie dazwischen liegendem Schrittbereich 16. Die Endbereiche 12 und 14 sind begrenzt durch Längsränder 18 und 20 sowie durch Querränder 22 und 24. Die Windel 10 kann in bekannter, nicht näher dargestellter Weise, aus einem flüssigkeitsdichten Wäscheschutz, einem darüber liegenden Saugkörper und einer den Saugkörper abdekkenden flüssigkeitsdurchlässigen Vliesstoffauflage gebildet sein. Entlang der Längsränder 18 und 20 und/oder der Querränder 22 und 24 können elastifizierte Seitenklappen vorgesehen sein, die die Dichtigkeit der Windel 10 beim Tragen erhöhen.

Um die Windel 10 an einer Person festzulegen, sind die Endbereiche 12 und 14 im Bereich der Längsränder 18 bzw. 20 miteinander verbindbar. Dazu sind erste Verschlußteile 26, die mit wenigstens einem zweiten Verschlußteil 28 in Eingriff bringbar sind, vorgesehen. Die ersten Verschlußteile 26 weisen eine erste Art mechanische Verschlußelemente 30, vorzugsweise Haken 32 auf. Wenigstens eines der ersten Verschlußteile 26 ist sowohl an dem Längsrand 18 als auch an dem Längsrand 20 im rückwärtigen Endbereich 14 befestigt.

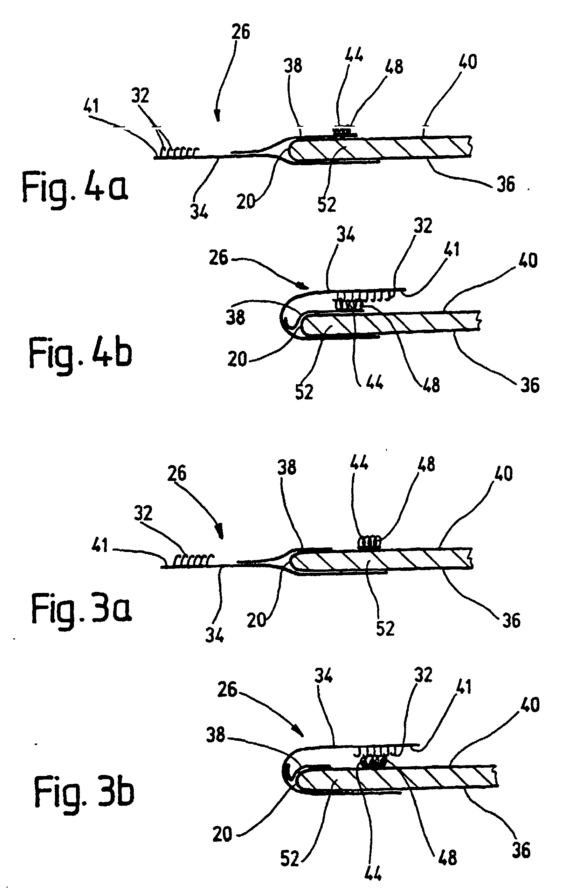

Das erste Verschlußteil 26 besteht dazu aus einem Streifen 34, der bevorzugt auf eine Außenseite 36 der Windel 10 beispielsweise durch Aufkleben oder Ultraschallverschweißen aufgebracht ist (Figur 3 und 4). Die Verbindung zwischen Windel 10 und erstem Verschlußteil 26 wird verstärkt durch ein Verankerungsteil 38, das einerseits bevorzugt auf eine Innenseite 40 der Windel 10 und andererseits auf den Streifen 34 aufgebracht ist. Auf seinem von der Windel 10 beabstandeten Ende weist der Streifen 34 die Haken 32 auf. Dabei ist das äußerste Ende des Streifens 34 frei von Haken, so daß eine Lasche 41 gebildet ist, an der das erste Verschlußteil 26 ergriffen werden kann, um es beispielsweise von dem zweiten Verschlußteil 28 zu lösen zum Öffnen der Windel 10.

Auf der Außenseite 36 der Windel 10 ist im vorderen Endbereich 12 das zweite Verschlußteil 28 befestigt (Figur 1). Das zweite Verschlußteil 12 weist eine zweite Art mechanischer Verschlußelemente 42, vorzugsweise Ösen 44, auf. Die Verschlußelemente der zweiten Art 42 sind mit den Verschlußelementen der ersten Art 30 lösbar in Eingriff bringbar nach Art eines Klettverschlusses, wodurch die Endbereiche 12 und 14 zum Festlegen der Windel 10 an einer Person verbindbar sind. Vorzugsweise erstreckt sich der zweite Verschlußteil 28 über die nahezu gesamte Breite der Windel 10, so daß die Windel 10 an Personen unterschiedlicher Größe fest anlegbar ist.

Die erfindungsgemäße Windel 10 weist auf ihrer Innenseite 40 in dem rückwärtigen Endbereich 14 wenigstens ein Halteelement 48 mit Verschlußelementen der zweiten Art 42, vorzugsweise Ösen 44 auf. Vorzugsweise ist jedem ersten Verschlußteil 26 ein Halteelement 48 zugeordnet. Dabei ist das Halteelement 48 mit einem Abstand vom zugeordneten Längsrand 18 bzw. 20 angeordnet, so daß das erste Verschlußteil 26 entlang des zugeordneten Längsrandes 18 bzw. 20 einfaltbar ist und mittels der Haken 32 an dem Halteelement 48 haftend in dieser auf die Windel 10 zurückgefalteten Konfiguration (Figur 3b und 4b) fixierbar ist. Das Halteelement 48 kann in seiner Ausdehnung sehr klein gehalten sein, denn es wirken auf das Halteelement 48 keine großen Kräfte. Das Halteelement 48 muß lediglich das erste Verschlußteil 26 in der eingefalteten Konfiguration halten können. Dies ist insbesondere in den Figuren 3b und 4b angedeutet. Dabei ist bevorzugt das Halteelement so angeordnet, daß die Einfaltung des Verschlußteils 26 in etwa bündig mit dem jeweiligen Längsrand 18 bzw. 20 ist.

Aufgrund der Sanduhrform der Windel 10 sind im rückwärtigen Endbereich 14 Seitenbereiche 50 und 52 gebildet, in denen die ersten Verschlußteile 26 und die zugeordneten Halteelemente 48 angeordnet sind. In einer Ausführungsform der Erfindung sind die Seitenbereiche 50 und 52 zumindest partiell elastifiziert, um einen optimalen Sitz an der Taille des Trägers zu gewährleisten. Um diese Elastizität durch Anbringen der ersten Verschlußteile nicht zu beeinträchtigen, bestehen der Streifen 34 und/oder das Verankerungsteil 38 wenigstens teilweise aus dehnbarem und/oder elastischem Material. Bevorzugt ist dann das Halteelement 48 mit Abstand von dem ersten Verschlußteil 26, insbesondere von dem Verankerungsteil 38, angeordnet, wie in den Figuren 1 bis 3 dargestellt.

In Figur 4a und b ist eine alternative Anordnung des Halteelements 48 gezeigt, bei der das Halteelement 48 auf dem Verankerungsteil 38 befestigt ist. Diese Anordnung empfiehlt sich aufgrund ihrer Einfachheit insbesondere dann, wenn die Seitenbereiche 50 nicht elastisch ausgebildet sind. Das Halteelement 48 kann dann zusammen mit dem Verankerungsteil 38 in einem Arbeitsgang auf die Windel aufgebracht werden.

Die erfindungsgemäße Windel wird wie folgt hergestellt: Zunächst wird in bekannter Weise der Saugkörper der Windel gefertigt und mit der flüssigkeitsdichten Wäscheschutzfolie auf der einen Seite und einer flüssigkeitsdurchlässigen Vliesstoffauflage auf der anderen Seite versehen, wodurch der Grundkörper der Windel erhalten ist. In einem nächsten Verfahrensschritt werden die ersten Verschlußteile 26 an der Windel 10 im Bereich der Längsränder 18 und 20 des rückwärtigen Endbereichs 14 befestigt. Dazu wird beispielsweise der Streifen 34 an der Wäscheschutzfolie (Außenseite 36) angeklebt oder angeschweißt. Um das Verschlußteil 26 sicher festzulegen, wird das Verankerungsteil 38 einerseits mit dem Streifen 34 und andererseits mit der flüssigkeitsdurchlässigen Vliesstoffauflage (Innenseite 40) verbunden. Dann wird das Halteelement 48 auf der Innenseite der Windel 10 durch Schweißen, Kleben oder ähnliches festgelegt.

Im nächsten Verfahrensschritt wird ein vom Längsrand abragender Abschnitt des ersten Verschlußteils umgeschlagen, so daß die Haken 32 des ersten Verschlußteils 26 mit den Ösen 44 des Halteelements 48 in Eingriff gelangen. Dadurch ist das erste Verschlußteil im weiteren Verfahrensablauf in auf die Windel 10 zurückgefalteter Konfiguration gehalten. Bevorzugt ist das Halteelement 48 so positioniert, daß die Einfaltung des Verschlußteils 26 in etwa bündig mit dem jeweiligen Längsrand 18 bzw. 20 ist.

Die Windel 10 wird dann in weiteren Verfahrensabschnitten endgefertigt und verpackt.

In Ausgestaltung des erfindungsgemäßen Verfahrens können einzelne Verfahrensabschnitte in ihrer zeitlichen Abfolge vertauscht werden. Beispielsweise können erst die Halteelemente 48 und dann die ersten Verschlußteile 26 befestigt werden oder umgekehrt. Auch ist es möglich, das Halteelement 48 und die ersten Verschlußteile 26 gleichzeitig an der Windel zu befestigen.

## Patentansprüche

1. Wegwerfwindel zum einmaligen Gebrauch mit einem vorderen und einem rückwärtigen Endbereich (12 und 14), die beide durch Längs- (18 und 20) und Querränder (22 und 24) der Windel (10) begrenzt sind, mit separaten ersten Verschlussteilen (26), die eine erste Art mechanische Verschlusselemente (30) aufweisen und von denen jeweils wenigstens eins an einem jeweiligen Längsrandbereich des rückwärtigen Endbereichs (14) befestigt ist und in Gebrauchsstellung von dem Längsrand (18 und 20) abragt, sowie mit einem zweiten Verschlussteil (28), das in dem vorderen Endbereich (12) befestigt ist, sich in Breitenrichtung der Windel unterbrechungsfrei durchgehend erstreckt und eine zweite Art mechanischer Verschlusselemente (42) aufweist, die mit den Verschlusselementen der ersten Art (30) lösbar in Eingriff bringbar sind, um die Endbereiche (12 und 14) zum Festlegen der Windel (10) an einer Person zu verbinden,
**dadurch gekennzeichnet, dass**
wenigstens ein Halteelement (48) mit Verschlusselementen der zweiten Art (42) in dem rückwärtigen Endbereich (14) angeordnet ist, um die ersten Verschlusselemente (26) zumindest bei der Herstellung der Windel (10) in auf die Windel (10) zurückgefalteter Konfiguration zu halten, und dass das jeweilige Halteelement (48) in einem solchen Abstand vom zugeordneten Längsrand (18, 20) positioniert ist, dass das dort vorgesehene erste Verschlussteil (26) entlang des zugeordneten Längsrands (18, 20) und in etwa bündig mit dem jeweiligen Längsrand (18, 20) auf die Windel einfaltbar und auf dem Halteelement fixierbar ist.

2. Wegwerfwindel nach Anspruch 1, **dadurch gekennzeichnet, daß** die erste Art mechanische Verschlußelemente (30) als Häkchen (32) und die zweite Art mechanische Verschlußelemente (42) als Ösen (44) nach Art eines Klettverschlusses ausgebildet sind.

3. Wegwerfwindel nach Anspruch 1 oder 2, **dadurch gekennzeichnet, daß** das Halteelement (48) auf einem Verankerungsteil (38) des ersten Verschlußteils (26) befestigt ist.

4. Wegwerfwindel nach Anspruch 1 oder 2, **dadurch gekennzeichnet, daß** das Halteelement (48) mit Abstand von dem ersten Verschlußteil (26) befestigt ist.

5. Wegwerfwindel nach einem der vorhergehenden Ansprüche, **dadurch gekennzeichnet, daß** die ersten Verschlußteile (26) wenigstens teilweise aus dehnbarem Material bestehen.

6. Wegwerfwindel nach einem der vorhergehenden Ansprüche, **dadurch gekennzeichnet, daß** die ersten Verschlußteile (26) wenigstens teilweise aus elastischem Material bestehen.

7. Wegwerfwindel nach einem der vorhergehenden Ansprüche, **dadurch gekennzeichnet, daß** die ersten Verschlußmittel (26) aus einem im Vergleich zum Material der Wegwerfwindel (10) formstabilen Material bestehen.

8. Verfahren zur Herstellung einer Wegwerfwindel nach einem der vorhergehenden Ansprüche 1 bis 7, bei dem in einem Verfahrensschritt die ersten Verschlußteile (26) an der Windel (10) im Bereich der Längsränder (18 und 20) des rückwärtigen Endbereichs (14) befestigt werden, so daß sie von dem jeweiligen Längsrand (18 bzw. 20) abragen, und bei dem ein zweites Verschlußteil (28) in Beitenrichtung der Windel unterbrechungsfrei durchgehend im Vordesheil (12) befestigt wird, **dadurch gekennzeichnet, daß** in einem weiteren Verfahrensschritt das Halteelement (48) mit Verschlußelementen der zweiten Art (42) in dem rückwärtigen Endbereich (14) befestigt wird und dann vom Längsrand (18, 20) abragende Abschnitte der ersten Verschlußteile (26) umgeschlagen werden und zumindest bereichsweise mit ihren Verschlußelementen der ersten Art (30) mit den Verschlußelementen der zweiten Art (42) des Halteelements (48) in Eingriff gelangen und so die ersten Verschlußteile (26) im weiteren Verfahrensablauf in auf die Windel (10) zurückgefalteter Konfiguration gehalten werden.

9. Verfahren nach Anspruch 8, **dadurch gekennzeichnet, daß** erst das Halteelement (48) und dann die ersten Verschlußteile (26) an der Windel (10) befestigt werden oder umgekehrt.

10. Verfahren nach Anspruch 8, **dadurch gekennzeichnet, daß** das Halteelement (48) im wesentlichen gleichzeitig mit den ersten Verschlußteilen (26) an der Windel befestigt wird.

## Claims

1. Single-use disposable nappy with a front and a back end region (12 and 14) which are both limited by longitudinal (18 and 20) and transverse edges (22 and 24) of the nappy (10) comprising separate first fastening parts (26) with a first type of mechanical fastening elements (30) and of which at least one in each case is fastened to a respective longitudinal edge region of the back end region (14) and in the position of use projects from the longitudinal edge (18 and 20), and comprising at least one second fastening part (28) fastened in the front end region (12) and having a second type of mechanical fastening elements (42) which can be detachably brought into engagement with the fastening elements of the first type (30) in order to connect the end regions (12 and 14) to fix the nappy (10) on a person, **characterised in that** at least one holding element (48) with fastening elements of the second type (42) is arranged in the back end region (14) in order to hold the first fastening elements (26), at least during production of the nappy (10), in a configuration folded back onto the nappy (10), and **in that** the respective holding element (48) is positioned at a distance from the associated longitudinal edge (18, 20) such that the first fastening part (26) provided there can be folded in onto the nappy along the associated longitudinal edge (18, 20) and approximately flush with the respective longitudinal edge (18, 20) and can be fixed to the holding element.

2. Disposable nappy according to claim 1, **characterised in that** the first type of mechanical fastening elements (30) are formed as small hooks (32) and the second type of mechanical fastening elements (42) are formed as eyes (44) in the manner of a burr-type fastener.

3. Disposable nappy according to claim 1 or 2, **characterised in that** the holding element (48) is fastened to an anchoring part (38) of the first fastening part (26).

4. Disposable nappy according to claim 1 or 2, **characterised in that** the holding element (48) is fastened at a distance from the first fastening part (26).

5. Disposable nappy according to any of the preceding claims, **characterised in that** the first fastening parts (26) consist at least partially of stretchable material.

6. Disposable nappy according to any of the preceding claims, **characterised in that** the first fastening parts (26) consist at least partially of elastic material.

7. Disposable nappy according to any of the preceding claims, **characterised in that** the first fastening means (26) consist of a dimensionally stable material in comparison with the material of the disposable nappy (10).

8. Method for producing a disposable nappy according to any of the preceding claims 1 to 7, the first fastening parts (26) being fastened to the nappy (10) in the region of the longitudinal edges (18 and 20) of the back end region (14) in one process step, such that they project from the respective longitudinal edge (18 and 20), **characterised in that** in a further process step the holding element (48) is fastened with fastening elements of the second type (42) in the back end region (14) and then portions of the first fastening parts (26) projecting from the longitudinal edge (18, 20) are folded back and, at least in certain regions, engage with their fastening elements of the first type (30) with the fastening elements of the second type (42) of the holding element (48) and therefore the first fastening parts (26) are held in the further process sequence in a configuration folded back onto the nappy (10).

9. Method according to claim 8, **characterised in that** first the holding element (48) and then the first fastening parts (26) are fastened to the nappy (10) or vice versa.

10. Method according to claim 8, **characterised in that** the holding element (48) is fastened to the nappy substantially simultaneously with the first fastening parts (26).

## Revendications

1. Couche jetable à usage unique qui comprend une zone terminale avant et une zone terminale arrière (12 et 14), qui sont toutes les deux limitées par des bords longitudinaux (18 et 20) et des bords transversaux (22 et 24) de la couche (10), des premières pièces de fermeture séparées (26), qui comportent un premier type d'éléments do fermeture mécanique (30) et parmi lesquelles au moins une est fixée à une zone de bord longitudinale respective de la zone terminale arrière (14) et fait saillie du bord longitudinal (18 et 20) en position d'utilisation, ainsi qu'une deuxième pièce de fermeture (28), qui est fixée dans la zone terminale avant (12). qui s'étend en continu sans interruption dans la direction de la largeur de la couche et qui comporte un deuxième type d'éléments de fermeture (42) qui pouvent être amenés en accrochage, de manière amovible. avec les éléments de fermoturo du premier type (30) pour relier les zones terminales (12 et 14) pour fixer la couche (10) sur une personne,
**caractérisée en ce que**
au moins un élément de maintien (48) est disposé, avec les éléments de fermeture du deuxième type (42), dans la zone terminale arriére (14) pour maintenir les premiers éléments de fermeture (26). pour le moins lors de la fabrication de la couche (10), dans une configuration repliée sur la couche (10) et **en ce que** l'élément de maintien (46) respectif est positionné à une distance du bord longitudinal associé (18, 20) qui est telle que la première pièce de fermeture (26) qui y est prévue peut Etre, le long du bord longitudinal associé (18, 20) of sonsiblement en affleurement avec le bord longitudinal (18, 20), repliée sur la couche et fixée sur l'élément de maintien.

2. Couche jetable selon la revendication 1, **caractérisée en ce que** le premier type des éléments de fermeture (30) est réalisé sous la forme de petits crochets (32) et le deuxième type des éléments de fermeture (42) sous la forme d'oeillets (44) à la manière d'une fermeture à glissière.

3. Couche jetable selon la revendication 1 ou 2, **caractérisée en ce que** l'élément de maintien (48) est fixé sur une pièce d'ancrage (38) de la première pièce de fermeture (26).

4. Couche jetable selon la revendication 1 ou 2, **caractérisée en ce que** l'élément de maintien (48) est fixé à distance de la première pièce de fermeture (26).

5. Couche jetable selon l'une quelconque des revendications précédentes, **caractérisée en ce que** les premières pièces de fermeture (26) sont constituées au moins partiellement de matière extensible.

6. Couche Jetable selon l'une quelconque des revendications précédentes, **caractérisée en ce que** les premières pièces de fermeture (20) sont constituées au moins partiellement de matiére élastique.

7. Couche Jetable selon l'une quelconque des revendications précédentes, **caractérisée en ce que** les premlers moyens de fermeture (26) sont constitués d'une matière indéformable par rapport à la matière de la couche jetable (10).

8. Procédé pour la fabrication d'un couche jetable selon l'une quelconque des revendications précédentes 1 à 7, dans lequel, dans une étape de procédé, les premières pièces de fermeture (26) sont fixées sur la couche (10) dans la zone des bords longitudinaux (18 et 20) de la zone terminale arrière (14) de telle manière qu'elles fassent saillie du bord longitudinal respectif (18 ou 20) et dans lequel une deuxième pièce de fermeture (28) est fixée, en continu sans interruption dans la direction de la largeur de la couche, dans la zone avant (12), **caractérisé en ce que**, dans une deuxième étape de procédé, l'élément de maintien (48) avec les éléments de fermeture du deuxième type (42) est fixé dans la zone terminale arriére (14) et **en ce que**, ensuite, des sections des premières pièces de fermeture (26) qui font saillie du bord longitudinal (18, 20) sont rabattues et viennent en accrochage, pour le moins par zones, par leurs éléments de fermeture du premier type (30), avec les éléments de fermeture du deuxième type (42) de l'élément do maintien (48) et, ainsi, les premières pièces de fermeture (26) sont, dans la suite du déroulement du procédé, maintenues dans la configuration repliée sur la couche (10).

9. Procédé selon la revendication 8, **caractérisé en ce que** l'élément de maintien (48) et, ensuite, les premières pièces do formoture (26) sont fixés sur la couche (10) ou Inversement.

10. Procédé selon la revendication 8, **caractérisé en ce que** l'élément de maintien (48) est fixé sensiblement en môme temps que les premières pièces de fermeture (26) sur la couche.
